Europäisches Patentamt

**European Patent Office**

Office européen des brevets

(19)

(11) Veröffentlichungsnummer: **0 021 424**
**B1**

(12) **EUROPÄISCHE PATENTSCHRIFT**

(45) Veröffentlichungstag der Patentschrift:
13.04.83

(21) Anmeldenummer: 80103601.3

(22) Anmeldetag: 25.06.80

(51) Int. Cl.³: **A 61 K 9/06**, A 61 K 33/18

(54) Jodidhaltiges Mittel zur Behandlung von Trübungen im Bereich des Auges.

(30) Priorität: 26.06.79 DE 2925804

(43) Veröffentlichungstag der Anmeldung:
07.01.81 Patentblatt 81/1

(45) Bekanntmachung des Hinweises auf die Patenterteilung:
13.04.83 Patentblatt 83/15

(84) Benannte Vertragsstaaten:
BE CH DE FR LI LU NL

(56) Entgegenhaltungen:
**SOVIET INVENTIONS ILLUSTRATED, Section Ch: Chemical, Woche B22; ausgegeben am 11. Juli 1979, Seite 14, Derwent Publications, London)**
**MANUFACTURING CHEMIST, Band 32, Nr. 10, Oktober 1961, Seite 469 »Pharmaceutical Formulation Problems«**
**CHEMICAL ABSTRACTS, Band 76, Nr. 22, 29. Mai 1972, Seite 310, Nr. 131 442n Columbus, Ohio, USA V. HENSCHEL: »Formulation and stabilization of magistral collyria«**
**LEBEAU P., G. COURTOISE: »Traité de Pharmacie Chimique«, III. Ausgabe, Band I »Chimie Mineral« 1946. Janot M., Guérin H., Corriez P., Seite 116 Paris, FR.**

(73) Patentinhaber: Kanoldt Arzneimittel GmbH,
Postfach 1160, D-8884 Höchstädt/Donau (DE)

(72) Erfinder: Gauri, Kailash Kumar, Dr., D-2359 Lentföhrden (DE)

(74) Vertreter: Kinzebach, Werner, Dr. Patentanwälte et al, Reitstötter J. Prof.Dr.Dr. Kinzebach W. Dr. & Partner Bauerstrasse 22 Postfach 780, D-8000 München 43 (DE)

DIE PHARMAZIE, Band 34, Nr. 2, Februar 1979, Seiten 119—125 Berlin, DE. H. WEISS: »Inkompatibilität und Instabilität bei Augenarzneien« Remington, Pharmaceutical Sciences, Seite 1178 (1975)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Jodidhaltiges Mittel zur Behandlung von Trübungen im Bereich des Auges

Die Erfindung betrifft ein wäßriges, natriumthiosulfat- und kaliumjodidhaltiges Mittel zur Behandlung von Trübungen im Bereich des Auges.

Soviet Invent. illustr., Sect. Ch: Chemical, 11.07.1979 beschreibt auf Seite 14 eine 3%ige Kaliumjodid-Lösung für medizinische Zwecke, die durch Zugabe von 0,001% Natriumthiosulfat chemisch stabilisiert wird.

In Manufacturing Chemist, 32, seite 469, ist die Zusammensetzung einer Salbe mit einem Gehalt von 20% Kaliumjodid angegeben, wobei ein Zusatz von 0,2% Natriumthiosulfat nur den Zweck hat, eine Braunfärbung der Salbe zu verhindern.

Es sind bereits eine Reihe von jodidhaltigen Präparaten anderer Zusammensetzung als das erfindungsgemäße zur Behandlung von Trübungserscheinungen im Bereich der Linse und des Glaskörpers des Auges bekannt, deren Wirkung jedoch recht umstritten ist.

Man nimmt aufgrund neuerer Untersuchungen an, daß die Trübungen im Bereich der Linse im wesentlichen darauf zurückzuführen sind, daß die freien SH-Funktionen der Linsen-Proteine über S-S-Brücken vernetzen, wodurch ein Teil der Transparenz des Linsenkörpers verlorengeht, (vgl. Harding, J. J. and K. J. Dilley, exp. Eye Res. 22, 1−73 [1976]).

Neurere Untersuchungen von Gauri (Gauri, K. K., Der Augenspiegel, Heft 2, 1979, Seiten 2 bis 7) deuten darauf hin, daß die Anwesenheit von Jod im Auge die Linsen-Proteine günstig beeinflußt, so daß die Bildung von Quer-Vernetzungen erschwert oder gar verhindert wird. Aufgrund der Fähigkeit von Jod, seinen Wertigkeitszustand rasch und leicht zu wechseln, kann man davon ausgehen, daß Jod nicht nur die Entstehung von Disulfid-Brücken verhindert sondern auch bereits gebildete Disulfid-Brücken wieder spalten kann. Folglich dürfte Jod die Katarakt-Entstehung prophylaktisch verhindern und zu einem gewissen Grad auch die bereits entstandenen Trübungen zurückbilden können.

Voraussetzung für eine derartige Wirkung von Jodid-Lösungen, ist eine ausreichende Bioverfügbarkeit von Jodionen bzw. Jod im Bereich der Augenlinse. Untersuchungen mit radioaktiv markiertem Jod am Kaninchenauge haben gezeigt, daß bei der Verwendung von wäßrigen Kaliumjodidhaltigen Lösungen die in der Linse anzutreffende Jodkonzentration zu gering ist, um eine therapeutische Wirkung verursachen zu können. Daraus erklärt sich auch die umstrittene Wirkung der bekannten jodhaltigen Mittel zur Behandlung von Trübungen im Bereich des Auges.

Überraschenderweise wurde nun gefunden, daß ein Zusatz von mehr als 0,5 bis maximal etwa 2 Gew.-% an Natriumthiosulfat zu 0,5 bis 2 Gew.-%igen, wäßrigen Kaliumjodidlösungen die Bioverfügbarkeit von Jod im Bereich der Linse erheblich steigert.

Die mit $J^{125}$ durchgeführten Versuche in Anwesenheit von 1 Gew.-% Thiosulfat in wäßriger Lösung führten zu folgender Aktivitätsverteilung im Auge:

| Augenbestandteil | Prozent der im Auge befindlichen Aktivität |
|---|---|
| Hornhaut | 41,0 |
| Kammerwasser | 47,0 |
| Linse | 6,4 |
| Glaskörper | 3,7 |
| Netzhaut | 1,8 |

Obige Tabelle zeigt die Verteilung der $^{125}$Jod-Isotope nach Einträufelung von jeweils 0,5 ml KJ-Zubereitung in Abständen von 15 Minuten (insgesamt 11 Einträufelungen). Entnahme der Gewebe 30 Minuten nach der letzten Behandlung. Ausgangsaktivität in 0,5 ml KI-Lösung $= 1,1 \cdot 10^9$ cpm.

Insgesamt gefunden im geträufelten Auge $= 9,99 \cdot 10^6 = 0,91\%$ der Ausgangsaktivität.

Zum Vergleich wurde eine einfache wäßrige KI-Lösung ohne erfindungswesentlichen Zusatz gleicher Aktivität und Menge eingeträufelt, dabei wurde nur 0,01% der Ausgangsaktivität im Auge gefunden.

Aufgrund dieser Ergebnisse wurden Versuche mit menschlichen Katarakt-Linsen durchgeführt. Derartige Linsen wurden bei 37°C in $Na_2S_2O_3$-haltigen wäßrigen Lösungen, die 0,014 bis 1% Kaliumjodid enthielten 12 Tage lang aufbewahrt. Dabei wurde eine aufhellende Wirkung in Abhängigkeit von der Zeit der Einwirkung und der Konzentration gefunden. Je höher die Konzentration in den hier beschriebenen Grenzen, desto rascher trat der aufhellende Effekt ein.

Vorzugsweise verwendet man den Natriumthiosulfatzusatz in einer Menge von 1 Gew.-%.

Die Erfindung betrifft somit ein natriumthiosulfat- und kaliumjodidhaltiges Mittel zur Behandlung von Trübungen vom Bereich des Auges, das dadurch gekennzeichnet ist, daß es in einem üblichen wäßrigen Träger 0,5 bis 2 Gew.-% Kaliumjodid und mehr als 0,5 bis maximal 2 Gew.-% Natriumthiosulfat enthält.

Erfindungswesentlich ist somit die Verwendung von mehr als 0,5 bis maximal 2,0 Gew.-% Natriumthiosulfat in Kombination mit 0,5 bis 2 Gew.-% Kaliumjodid zur Herstellung wäßriger Lösungen zur Behandlung von Trübungen im Bereich des Auges.

## Patentanspruch

Wäßriges, natriumthiosulfat- und kaliumjodid-haltiges Mittel zur Behandlung von Trübungen im Bereich des Auges dadurch gekennzeichnet, daß der Gehalt an Natriumthiosulfat mehr als 0,5 bis maximal 2 Gew.-% und der Gehalt an Kaliumjodid 0,5 bis 2 Gew.-% beträgt.

## Claim

Aqueous composition containing sodium thiosulfate and potassium iodide, for treating opaque areas in the eye, characterized in that the content of sodium thiosulfate is more than 0.5 to a maximum of 2% by weight and the content of potassium iodide is 0.5 to 2% by weight.

## Revendication

Composition aqueuse contenant du thiosulfate de sodium et de l'iodure de potassium pour traiter des opacités dans les yeux, caractérisée en ce que le content en thiosulfate de sodium est plus que 0,5 jusqu'à un maximum de 2% et que le content en iodure de potassium est de 0.5 jusqu'à 2% en poids.